# EUROPEAN PATENT APPLICATION

(11) **EP 0 775 489 A1**
(43) Date of publication of application: **28.05.1997**
(21) Application number: 95928005.8
(22) Date of filing: 10.08.1995
(51) Int. Cl.: A61K 31/70, C07H 17/08

(54) **INTERLEUKIN-5 PRODUCTION INHIBITOR**

(30) Priority: 12.08.1994 JP 190289/94; 12.08.1994 JP 190292/94
(71) Applicant: TAISHO PHARMACEUTICAL CO. LTD, Tokyo 171 (JP)
(72) Inventor: HOSHINO, Akihiko Taisho Pharmaceutical Co., Ltd., Tokyo 171 (JP); KASHIMURA, Masato Taisho Pharmaceutical Co., Ltd., Tokyo 171 (JP); ASAKA, Toshifumi Taisho Pharmaceutical Co., Ltd., Tokyo 171 (JP); INOUE, Tomoyuki Taisho Pharmaceutical Co., Ltd., Tokyo 171 (JP); OKUDAIRA, Hirokazu, Tokyo 112 (JP)
(74) Representative: Ruffles, Graham Keith
(86) International application number: JP9501592
(87) International publication number: WO9604920

(57) **Abstract**

Object: to provide an erythromycin derivative having a potent interleukin-5 production inhibitory effect. Constitution: an interleukin-5 production inhibitor containing as the active ingredient an erythromycin derivative, such as 6-O-methylerythromycin A 9-[O-(2-chlorophenylmethyl)oxime], 6-O-methylerythromycin A 9-[O-(3-methoxy-4-t-butylbenzyl)oxime] 11,12-cyclic carbonate or 11-amino-9-N, 11-N-cyclic ethylene-9-deoxo-11-deoxy-6-O-methylerythromycin A 9-benzylamine 11-N, 12-O-cyclic carbamate, or a medicinally acceptable acid-addition salt thereof.

## Description

### Technical Field

This invention relates to an inhibitor on interleukin 5 production which contains an erythromycin derivative as an active ingredient.

### Background Art

Interleukin 5 (hereinafter abbreviated as IL-5) is known to be an important factor stimulating differentiation and growth of eosinophils which accelerate allergic inflammation. Therefore, an inhibitor on IL-5 production is useful for the treatment of various allergic diseases, such as bronchial asthma, allergic rhinitis, atopic dermatitis, drug allergy, and eosinophilic pneumonia.

Erythromycin is an antibiotic that has been of wide clinical use as a treating agent for infections caused by Gram-positive bacteria, some kinds of Gram-negative bacteria, Mycoplasma, etc. It has recently been reported that erythromycin and roxithromycin have an inhibitory activity on IL-5 production (Japanese Journal of Allergology, 44(3-2), p. 424 (1993)), but the IL-5 production inhibitory activity of erythromycin and roxithromycin is not sufficient.

An object of this invention is to provide an erythromycin derivative having a potent IL-5 production inhibitory activity.

### Disclosure of Invention

The inventors of the present invention have extensively studied the IL-5 inhibitory activity of erythromycin derivatives, and as a result, have found that the following erythromycin derivatives exhibit a potent IL-5 production inhibitory activity and thus completed the present invention.

The present invention provides an IL-5 production inhibitor comprising 6-O-methylerythromycin A 9-[O-(2-chlorophenylmethyl)oxime], 6-O-methylerythromycin A 9-[O-(3-methoxy-4-t-butylbenzyl)oxime] 11,12-cyclic carbonate, 11-amino-9-N,11-N-cyclic ethylene-9-deoxo-11-deoxy-6-O-methylerythromycin A 9-benzylamine 11-N,12-O-cyclic carbamate, or a pharmaceutically acceptable acid addition salt thereof as an active ingredient.

The pharmaceutically acceptable acid addition salt for use in the invention includes an acetate, a propionate, a butyrate, a formate, a trifluoroacetate, a maleate, a tartrate, a citrate, a stearate, a succinate, an ethylsuccinate, a lactobionate, a gluconate, a glucoheptonate, a benzoate, a methanesulfonate, an ethanesulfonate, a 2-hydroxyethanesulfonate, a benzenesulfonate, a p-toluenesulfonate, a laurylsulfate, a malate, an aspartate, a glutamate, an adipate, a cysteinate, a hydrochloride, a hydrobromide, a phosphate, a sulfate, a hydroiodide, a nicotinate, an oxalate, a picrate, a thiocyanate, an undecanoate, a salt of an acrylic acid polymer, and a salt of a carboxyvinyl polymer.

The 6-O-methylerythromycin A 9-[O-(2-chlorophenylmethyl)oxime] and 6-O-methylerythromycin A 9-[O-(3-methoxy-4-t-butylbenzyl)oxime] 11,12-cyclic carbonate used in the invention can be prepared, for example, as follows.
Step (1): 6-O-Methylerythromycin A 9-oxime (described in U.S. Patent 4,680,386) dissolved in an inert solvent is reacted with 2-chlorobenzyl chloride in the presence of a base to prepare 6-O-methylerythromycin A 9-[O-(2-chlorophenylmethyl)oxime]. Suitable inert solvents include acetone, tetrahydrofuran, N,N-dimethylformamide, dimethyl sulfoxide, dioxane, and mixtures thereof. Suitable bases include sodium hydride, sodium hydroxide, and potassium hydroxide. The reaction temperature ranges from -20° to 50°C, preferably 0° to 25°C.
Step (2): Step (1) as described above is repeated, except for replacing 2-chlorobenzyl chloride with 3-methoxy-4-t-butylbenzyl bromide. The resulting compound is reacted with acetic anhydride or acetyl chloride to acetylate at the 2'-position. The resulting compound is reacted with such a reagent as a phosgene dimer or trimer in an inert solvent in the presence of a base, such as pyridine, under cooling with ice, followed by deacetylating at the 2'-position to obtain 6-O-methylerythromycin A 9-[O-(3-methoxy-4-t-butylbenzyl)oxime) 11,12-cyclic carbonate. Suitable inert solvents include acetone, ethyl acetate, dichloromethane, tetrahydrofuran, acetonitrile, and N,N-dimethylformamide. The reaction temperature ranges from -20° to 50°C, preferably 0° to 25°C.

The 11-amino-9-N,11-N-cyclic ethylene-9-deoxo-11-deoxy-6-O-methylerythromycin A 9-benzylamine 11-N,12-O-cyclic carbamate used in the invention can be prepared, for example, as follows.

11-Amino-9-N,11-N-cyclic ethylene-9-deoxo-11-deoxy-6-O-methylerythromycin A 9-imine 11-N,12-O-cyclic carbamate described in WO 92/09614 is reacted with a reducing agent in an appropriate solvent in the presence of an appropriate acid to obtain 11-amino-9-N,11-N-cyclic ethylene-9-deoxo-11-deoxy-6-O-methylerythromycin A 9-amine 11-N,12-O-cyclic carbamate, which is then reacted with formic acid and benzaldehyde in an appropriate solvent to prepare 11-amino-9-N,11-N-cyclic ethylene-9-deoxy-11-deoxy-6-O-methylerythromycin A 9-benzylamine 11-N,12-O-cyclic carbamate. Suitable solvents include methanol, ethanol, N,N-dimethylformamide, and mixtures thereof. Suitable acids include acetic acid and formic acid. Useful reducing agents include sodium borohydride and sodium borocyanide.

The compounds according to the invention can be administered orally or non-orally in the form of tablets, capsules, granules, dusts, powders, troches, ointments, creams, emulsions, suspensions, suppositories, and injectable solutions. These dose forms can be prepared in a conventional manner, for example, the methods specified in Japanese Pharmacopoeia (12th Rev.). An appropriate dose form is chosen depending on the conditions and age of a patient and the purpose of treatment. In the preparation of various dose forms, commonly employed vehicles (e.g., crystalline cellulose, starch, lactose, mannitol), binders (e.g., hydroxypropyl cellulose, polyvinylpyrrolidone), lubricants (e.g., magnesium stearate, talc), disintegrants (e.g., carboxymethyl cellulose calcium), and the like can be used.

The dosage of the compound of the present invention ranges, for example, from 50 to 2000 mg in 2 to 3-divided doses per day in oral administration to adults, while appropriately varying depending on the age, body weight and conditions of a patient.

### Industrial Applicability

The compounds according to the invention exhibit a potent IL-5 production inhibitory activity and are useful as an IL-5 production inhibitor in humans and animals (inclusive of livestock). Thus, the compounds of the invention are effective on diseases caused by IL-5 production, i.e., various allergic diseases, such as bronchial asthma, allergic rhinitis, atopic dermatitis, drug allergy, and eosinophilic pneumonia.

### Best Mode for Carrying out Invention

The present invention is now illustrated in greater detail with reference to Examples.

### Example 1: Preparation of 6-O-Methylerythromycin A 9-[O-(2-Chlorophenylmethyl)oxime]

In 80 ml of dioxane was dissolved 5.33 g (7 mmol) of 6-O-methylerythromycin A 9-oxime, and 1.69 g (10.5 mmol) of 2-chlorobenzyl chloride and 536 mg (9.1 mmol) of 95% potassium hydroxide were added to the solution under cooling with ice, followed by stirring at room temperature for 2 hours. The reaction mixture was extracted with ethyl acetate, and the extract was washed with a saturated sodium chloride aqueous solution, dried over anhydrous magnesium sulfate, and evaporated under reduced pressure to remove the solvent. The residue was purified by silica gel column chromatography (eluent: chloroform/methanol/aqueous ammonia= 19:1:0.1) to give 3.92 g of the title compound as a yellow foamy substance.
Melting point: 145-147°C
¹H-NMR (300 MHz, CDCl₃) δ ppm:
   1.43 (3H, s), 2.33 (6H, s), 3.01 (3H, s), 3.32 (3H, s), 5.10, 5.17 (2H, ABq, J=13.8Hz), 7.19-7.44 (4H, m)
¹³C-NMR (75 MHz, CDCl₃) δ ppm: 40.3, 49.5, 50.8, 171.1, 175.6
IR (KBr), cm⁻¹: 3431, 1734

### Example 2: Preparation of 6-O-Methylerythromycin A 9-[O-(3-Methoxy-4-t-butylbenzyl)oxime] 11,12-Cyclic Carbonate

(1) The same reaction as in Example 1 was repeated, except for using 95% potassium hydroxide and 3-methoxy-4-t-butylbenzyl bromide to obtain 4.25 g of 6-O-methylerythromycin A 9-[O-(3-methoxy-4-t-butylbenzyl)oxime] from 4.96 g of the starting material (6-O-methylerythromycin A 9-oxime).
   Mass (FAB) m/z: 939 [MH]⁺
(2) In 15 ml of acetone was dissolved 1.6 g (1.70 mmol) of the compound obtained in (1) above, and 0.32 ml (3.41 mmol) of acetic anhydride was added to the solution, followed by stirring at room temperature for 2 hours. The solvent was removed by evaporation under reduced pressure to give 1.8 g of a 2'-acetylated compound as a foamy substance. The resulting compound was dissolved in 20 ml of dichloromethane, and 2.06 ml (25.6 mmol) of pyridine and 0.51 ml (4.26 mmol) of trichloromethyl chloroformate were added thereto while cooling with ice, followed by stirring for 2.5 hours. To the reaction mixture were added ice and sodium hydrogencarbonate, and the mixture was post-treated in a usual manner. The solvent was removed by evaporation under reduced pressure. The residue was heated in methanol to deacetylate at the 2'-position to obtain 881 mg of the title compound.
   Mass (FAB) m/z: 965 [MH]⁺
   ¹H-NMR (200 MHz, CDCl₃) δ ppm:
      2.44 (6H, s), 2.85 (3H, s), 3.32 (3H, s), 3.85 (3H, s)
   IR (KBr) cm⁻¹: 3457, 2972, 1815, 1741, 1461

### Example 3: Preparation of 11-Amino-9-N,11-N-cyclic ethylene-9-deoxo-11-deoxy-6-O-methylerythromycin A 9-Benzylamine 11-N,12-O-Cyclic Carbamate

(1) In 200 ml of a mixed solvent of ethanol/N,N-dimethylformamide (1/1) was dissolved 12 g (15.1 mmol) of 11-amino-9-N,11-N-cyclic ethylene-9-deoxo-11-deoxy-6-O-methylerythromycin A 9-imine 11-N,12-O-cyclic carbamate, and 1.72 ml (30.0 mmol) of acetic acid and 3.78 g (60.2 mmol) of NaBH₃CN were added thereto, followed by heating under reflux for 4 hours. To the mixture was added 1.42 g (22.6 mmol) of NaBH₃CN, and heat-refluxing was continued for an additional 1.5 hour period. The solvent was removed by evaporation, and to the residue was added a 2N sodium hydroxide aqueous solution. The reaction mixture was extracted with ethyl acetate, and the organic layer was washed successively with water and a saturated sodium chloride aqueous solution, and dried over anhydrous sodium sulfate. The solvent was evaporated under reduced pressure, and the residue was recrystallized from ethyl acetate/dichloromethane to give 10.9 g of 11-amino-9-N,11-N-cyclic ethylene-9-deoxo-11-deoxy-6-O-methylerythromycin A 9-amine 11-N,12-O-cyclic carbamate.
(2) In 5 ml of ethanol was dissolved 1 g (1.25 mmol) of the compound obtained in (1) above; and 0.5 ml (4.92 mmol) of benzaldehyde and 0.1 ml (2.35 mmol) of 90% formic acid were added thereto, followed by heating under reflux for 1 hour. Thereafter, 0.8 ml (18.8 mmol) of 90% formic acid was added thereto in 4 divided portions, and heating was continued for 21 hours. After the reaction, the reaction mixture was post-treated in the same manner as in Example 1. The crude product was purified by silica gel column chromatography (chloroform/methanol/aqueous ammonia=20:1:0.1) and recrystallization from dichloromethane/n-hexane to give 329 mg of the title compound.
   Melting point: 141-143°C and 210-225°C
   Mass (FAB) m/z: 890 [MH]⁺
   ¹H-NMR (300 MHz, CDCl₃) δ ppm:
      2.29 [6H, 3'-N(CH₃)₂], 3.31 (3H, 6-OCH₃), 3.32 (3H, 3''-OCH₃), 4.04 (2H, NCH₂), 7.18-7.35 (5H, Ph)
   ¹³C-NMR (75 MHz, CDCl₃) δ ppm:
      40.3 [3'-N(CH₃)₂], 42.1 (NCH₂), 42.8 (NCH₂), 49.4 (3''-OCH₃), 50.9 (6-OCH₃), 62.1 (9-NCH₂), 127.0, 128.3, 129.0, 140.2 (Ph)

### Example 4:

Ten grams of the compound prepared in Example 1, 550 g of lactose, 300 g of corn starch, 100 g of carboxymethyl cellulose calcium, and 30 g of polyvinylpyrrolidone were mixed well, and the mixture was granulated using ethanol, dried and classified in a usual manner. The granules were mixed with 10 g of magnesium stearate, and the mixture was tableted in a usual manner to obtain tablets each weighing 100 mg.

### Example 5:

The action on IL-5 production in mice was examined according to the following method as disclosed in M. Hikida, et al., Immunology Letters, Vol. 34, pp. 297-302 (1992).

Murine Th2 clone (D10.G4.1 cells) was purchased from ATCC. Antigen presenting cells were prepared by suspending 1 x 10⁷ spleen cells of a 8-week-old female C3H/HeN mice in 5 ml of an RPMI-1640 medium and incubated together with 50 µg/ml of mitomycin C (MMC) at 37°C for 30 minutes and then washed with three 50 ml portions of RPMI-1640. To RPMI-1640 containing 10% bovine serum were added 0.5 U of IL-2 (produced by Genzyme) and 5 x 10⁻⁵ M of 2-mercaptoethanol to prepare a medium for tissue culture. A test compound was dissolved in dimethyl sulfoxide (DMSO) and diluted with the tissue culture medium to have a final DMSO concentration of 0.1% and a varied test compound concentration.

In each well of a 96-well microtiter plate (produced by Corning Glass Works) were put each 50 µl/well of 4 x 10⁵ cells/ml of D10.G4.1 cells, 2 x 10⁶ cells/ml of MMC-treated antigen presenting cells, 400 µg/ml of conalbumin (produced by Sigma) as an antigen and the solution of the test compound in the tissue culture medium (to make 200 µl/well), and incubated in an incubator under 5% CO₂ at 37°C for 48 hours. After completion of the incubation, the supernatant liquor of the culture was collected, and the cells were separated by centrifugation. The IL-5 in the supernatant liquor was determined with an IL-5 ELISA kit produced by ENDOGEN. The inhibitory effect of the test compound on IL-5 production, expressed in terms of 50% inhibitory concentration (IC₅₀), is shown in Table 1 below.

**TABLE 1**

| Test Compound | IC₅₀ (M) |
|---|---|
| Compound of Example 1 | 2.7 x 10⁻⁷ |
| Compound of Example 2 | 1.2 x 10⁻⁷ |
| Compound of Example 3 | 1.2 x 10⁻⁷ |
| Roxithromycin | 9.3 x 10⁻⁷ |
| Erythromycin | 4.5 x 10⁻⁶ |

### Example 6:

The compounds of Examples 1, 2, and 3 were orally administered to ICR male mice grouped in fives. No death was observed at a dose level of 100 mg/kg, proving that the compounds are of high safety.

## Claims

1. An interleukin 5 production inhibitor comprising 6-O-methylerythromycin A 9-[O-(2-chlorophenylmethyl)oxime], 6-O-methylerythromycin A 9-[O-(3-methoxy-4-t-butylbenzyl)oxime] 11,12-cyclic carbonate, 11-amino-9-N,11-N-cyclic ethylene-9-deoxo-11-deoxy-6-O-methylerythromycin A 9-benzylamine 11-N,12-O-cyclic carbamate, or a pharmaceutically acceptable acid addition salt thereof as an active ingredient.

2. Use of 6-O-methylerythromycin A 9-[O-(2-chlorophenylmethyl)oxime], 6-O-methylerythromycin A 9-[O-(3-methoxy-4-t-butylbenzyl)oxime] 11,12-cyclic carbonate, 11-amino-9-N,11-N-cyclic ethylene-9-deoxo-11-deoxy-6-O-methylerythromycin A 9-benzylamine 11-N,12-O-cyclic carbamate, or a pharmaceutically acceptable acid addition salt thereof for the production of an interleukin 5 production inhibitor.

3. A method for treating an allergic disease comprising administering 6-O-methylerythromycin A 9-[O-(2-chlorophenylmethyl)oxime], 6-O-methylerythromycin A 9-[O-(3-methoxy-4-t-butylbenzyl)oxime] 11,12-cyclic carbonate, 11-amino-9-N,11-N-cyclic ethylene-9-deoxo-11-deoxy-6-O-methylerythromycin A 9-benzylamine 11-N,12-O-cyclic carbamate, or a pharmaceutically acceptable acid addition salt thereof.
